# EUROPEAN PATENT APPLICATION

(11) **EP 4 692 358 A1**
(43) Date of publication of application: **11.02.2026**
(21) Application number: 24781130.0
(22) Date of filing: 22.03.2024
(51) Int. Cl.: C12N 15/77, C12N 9/02, C12N 9/04, C12N 15/52, C12P 7/18

(54) **MUTATED MICROORGANISM FOR PRODUCING 1,4-BUTANEDIOL, AND METHOD FOR PRODUCING 1,4-BUTANEDIOL USING SAME**

(30) Priority: 31.03.2023 KR 20230043047; 21.03.2024 KR 20240038864
(71) Applicant: Daesang Corporation, Seoul 02586 (KR)
(72) Inventor: PARK, Si Jae, Goyang-si Gyeonggi-do 10234 (KR); SOHN, Yu Jung, Seoul 03780 (KR); JEONG, Seon A, Ulsan 44603 (KR); JOO, Jeong Chan, Seoul 04076 (KR); KIM, Moon Jeong, Seoul 07789 (KR); LEE, Jae Hun, Seoul 07789 (KR); YANG, Young Lyeol, Seoul 07789 (KR)
(74) Representative: Ipsilon
(86) International application number: PCT/KR2024/003608
(87) International publication number: WO 2024/205139

(57) **Abstract**

The present invention relates to a mutant microorganism for producing 1,4-butanediol and a method of producing 1,4-butanediol using same. Specifically, the mutant microorganism for producing 1,4-butanediol not only has excellent 1,4-butanediol production efficiency and productivity, but also generates less by-products during the production process, and thus may solve the problem of yield reduction caused by side-reaction products.

## Description

### Technical Field

The present invention relates to a mutant microorganism for producing 1,4-butanediol and a method of producing 1,4-butanediol using the same.

### Background Art

1,4-butanediol is a high value-added intermediate material that is used in the production of fibers, plastics, electronic chemicals, etc. In particular, it is in high demand worldwide as it is an important raw material for the production of solvents such as tetrahydrofuran (THF), polyurethane, elastic fibers, etc.

Industrial production of 1,4-butanediol through chemical reactions presents problems, including reduced yields due to side-reaction products, high energy consumption, and the need for high temperatures and pressures. Therefore, biotechnological production of 1,4-butanediol has emerged as a promising, low-cost, and sustainable alternative to chemical synthesis.

The first reported 1,4-butanediol biosynthetic metabolic pathway was constructed from glucose using succinyl-CoA as a precursor (Non-Patent Document 1), and since then, production of 1,4-butanediol using succinyl-CoA as a precursor was reported through metabolic engineering improvement of *E. coli* (Non-Patent Document 2). Recently, an example for the construction of a glutamate-based 1,4-butanediol-producing metabolic pathway in *E. coli* was reported. However, there was a limitation in that, when glucose and glutamate were supplied together, the final production of 1,4-butanediol in the recombinant *E. coli* was 1.21 g/L (Non-Patent Document 3), which was a low production yield. Therefore, to overcome this limitation, continuous efforts are needed to develop microorganisms, which are capable of producing 1,4-butanediol in high yields, using genetic engineering technology.

### DISCLOSURE

### Technical Problem

An object of the present invention is to provide a mutant microorganism for producing 1,4-butanediol, which generates less by-products while having excellent 1,4-butanediol productivity and production efficiency.

Another object of the present invention is to provide a method of producing 1,4-butanediol using the mutant microorganism.

### Technical Solution

An object of the present invention is to provide a mutant microorganism for producing 1,4-butanediol, which generates less by-products while having excellent 1,4-butanediol productivity and production efficiency.

One aspect of the present invention provides a mutant microorganism for producing 1,4-butanediol, which expresses: an enzyme that converts 4-hydroxybutyrate to 4-hydroxybutyraldehyde; and an enzyme that converts 4-hydroxybutyraldehyde to 1,4-butanediol.

According to one embodiment of the present invention, the mutant microorganism may be a *Corynebacterium glutamicum* mutant strain.

According to one embodiment of the present invention, the mutant strain may be transformed with a vector comprising: a gene encoding the enzyme that converts 4-hydroxybutyrate to 4-hydroxybutyraldehyde; and a gene encoding the enzyme that converts 4-hydroxybutyraldehyde to 1,4-butanediol.

According to one embodiment of the present invention, the enzyme that converts 4-hydroxybutyrate to 4-hydroxybutyraldehyde may be carboxylic acid reductase.

According to one embodiment of the present invention, the enzyme that converts 4-hydroxybutyrate to 4-hydroxybutyraldehyde may consist of the amino acid sequence of SEQ ID NO: 31.

According to one embodiment of the present invention, the enzyme that converts 4-hydroxybutyraldehyde to 1,4-butanediol may be aldehyde reductase YqhD or alcohol dehydrogenase YqhD.

According to one embodiment of the present invention, the enzyme that converts 4-hydroxybutyraldehyde to 1,4-butanediol may consist of the amino acid sequence of SEQ ID NO: 32.

According to one embodiment of the present invention, the mutant strain may be further express at least one of: an enzyme that converts glutamate to 4-aminobutyric acid; an enzyme that converts 4-aminobutyric acid to succinate semialdehyde; and an enzyme that converts succinate semialdehyde to 4-hydroxybutyrate.

According to one embodiment of the present invention, the mutant strain may be transformed with the vector further comprising at least one of: a gene encoding an enzyme that converts glutamate to 4-aminobutyric acid; a gene encoding an enzyme that converts 4-aminobutyric acid to succinate semialdehyde; and a gene encoding an enzyme that converts succinate semialdehyde to 4-hydroxybutyrate.

According to one embodiment of the present invention, the enzyme that converts glutamate to 4-aminobutyric acid may be glutamate decarboxylase.

According to one embodiment of the present invention, the enzyme that converts 4-aminobutyric acid to succinate semialdehyde may be 4-aminobutyrate aminotransferase.

According to one embodiment of the present invention, the enzyme that converts the succinate semialdehyde to 4-hydroxybutyrate may be aldehyde reductase YqhD or alcohol dehydrogenase YqhD.

According to one embodiment of the present invention, the mutant strain may be deficient in at least one of gabD gene, ldhA gene, and poxB gene.

Another object of the present invention is to provide a method of producing 1,4-butanediol using the mutant microorganism.

Another aspect of the present invention provides a method for producing 1,4-butanediol, comprising steps of: culturing the above-described *Corynebacterium glutamicum* mutant strain; and recovering 1,4-butanediol from the mutant strain or a medium in which the mutant strain has been cultured.

### Advantageous Effects

The mutant microorganism for producing 1,4-butanediol according to the present invention not only has excellent 1,4-butanediol production efficiency and productivity, but also generates less by-products during the production process, and thus may solve the problem of yield reduction caused by side-reaction products.

### Brief Description of Drawings

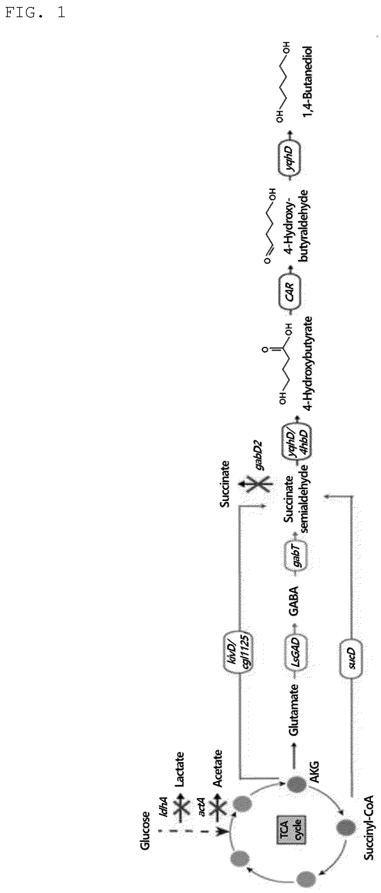

FIG. 1 shows a CoA-independent 1,4-butanediol production pathway.

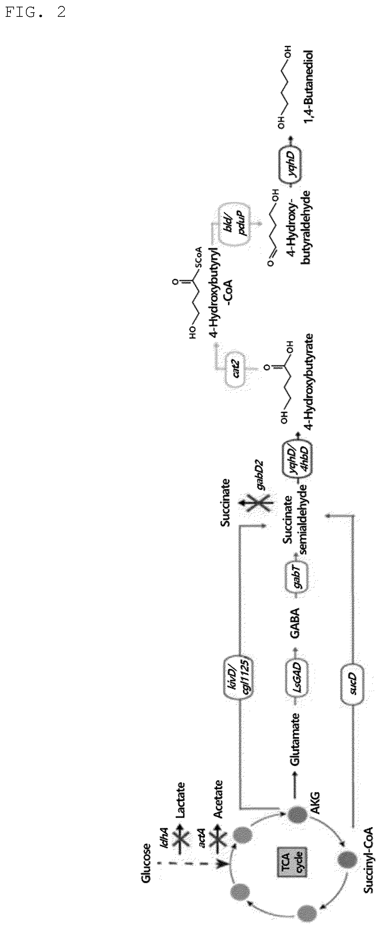

FIG. 2 shows a CoA-dependent 1,4-butanediol production pathway.
FIG. 3 shows various metabolic pathways for producing 4-hydroxybutyrate, a key precursor of 1,4-butanediol.
FIG. 4 is a graph showing the amount of 4-hydroxybutyrate (4HB) produced in C. *glutamicum ΔgabD2* transformed with each plasmid constructed for each metabolic pathway for production of 4HB in Example 1.
FIG. 5 is a graph showing the results of comparing the CoA-dependent pathway and the CoA-independent pathway and screening 4HB conversion systems according to Example 2, in terms of the amount of 1,4-butanediol (14BDO) produced.
FIG. 6 is a graph comparing the 14BDO productivity of recombinant strains transformed to use the CoA-dependent pathway.
FIG. 7 is a graph comparing the 14BDO productivity of recombinant strains transformed to use the CoA-independent pathway.

### Best Mode

One aspect of the present invention provides a mutant microorganism for producing 1,4-butanediol, which expresses: an enzyme that converts 4-hydroxybutyrate to 4-hydroxybutyraldehyde; and an enzyme that converts 4-hydroxybutyraldehyde to 1,4-butanediol.

The "mutant microorganism for producing 1,4-butanediol" as used in the present invention is a microorganism that expresses an enzyme involved in the 1,4-butanediol production pathway or a gene encoding the enzyme, and refers to a modified microorganism obtained by modifying an endogenous gene sequence (nucleic acid deletion, addition, substitution, etc.) for the gene encoding the relevant enzyme, inserting a foreign gene into the genome, and/or introducing a plasmid (vector) containing a foreign gene. In the present invention, the term "modified microorganism" may be used interchangeably with the term "mutant microorganism", "mutant strain", "recombinant microorganism", "recombinant strain", "mutated strain", "genetically modified microorganism", or "genetically modified strain". In contrast, the term "microorganism before modification", "strain before modification", "non-mutated microorganism", "non-mutated strain", or "parent strain" refers to a microorganism before the trait thereof is changed by genetic mutation due to natural or artificial factors, for example, a microorganism that does not/cannot produce 1,4-butanediol due to the lack of enzymes that acts in the 1,4-butanediol production pathway.

According to one embodiment of the present invention, the mutant microorganism may be a *Corynebacterium glutamicum* mutant strain.

According to one embodiment of the present invention, the mutant strain may be transformed with a vector comprising: a gene encoding the enzyme that converts 4-hydroxybutyrate to 4-hydroxybutyraldehyde; and a gene encoding the enzyme that converts 4-hydroxybutyraldehyde to 1,4-butanediol.

As used in the present invention, the term "vector" refers to any type of nucleic acid sequence transfer structure that is used as a means for transferring and expressing a gene of interest in a host cell. Unless otherwise particularly specified, the term "vector" may mean one allowing the nucleic acid sequence contained therein to be expressed after insertion into the host cell genome and/or one allowing the nucleic acid sequence to be expressed independently. This vector comprises essential regulatory elements operably linked so that an inserted gene can be expressed. The term "operably linked" means that a gene of interest and regulatory sequences thereof are functionally linked together in a manner enabling gene expression, and the "regulatory elements" include a promoter for initiating transcription, any operator sequence for regulating transcription, a sequence encoding suitable mRNA ribosome-binding sites, and a sequence for regulating termination of transcription and translation.

The vector that is used in the present invention is not particularly limited as long as it is replicable in a host cell, and any vector known in the art may be used. Examples of the vector include a natural or recombinant plasmid, cosmid, virus and bacteriophage. Examples of a phage vector or cosmid vector include, but are not limited to, pWE15, M13, AMBL3, AMBL4, λIXII, λASHII, AAPII, At10, λt11, Charon4A, and Charon21A, and examples of a plasmid vector include, but are not limited to, pBR series, pUC series, pBluescriptII series, pGEM series, pTZ series, pCL series, and pET series.

The vector may typically be constructed as a vector for cloning or as a vector for expression. The vector for expression may be a conventional vector that is used in the art to express a foreign gene or protein in a plant, animal, or microorganism, and may be constructed through various methods known in the art.

The "recombinant vector" used in the present invention may be constructed using a prokaryotic or eukaryotic cell as a host, and may replicate regardless of the genome of the host cell or may be integrated into the genome itself. The host cell may contain a replication origin, which is a particular nucleotide sequence which enables the vector to replicate in the host cell and from which replication starts. For example, when the vector used is an expression vector and uses a prokaryotic cell as a host, the vector generally comprises a strong promoter capable of promoting transcription (e.g., pLλ promoter, CMV promoter, trp promoter, lac promoter, tac promoter, T7 promoter), a ribosome binding site for initiation of translation, and a transcription/translation termination sequence. When a eukaryotic cell is used as a host, the vector comprises a replication origin operating in the eukaryotic cell, and examples of the replication origin include, but are not limited to, an f1 replication origin, an SV40 replication origin, a pMB1 replication origin, an adeno replication origin, an AAV replication origin, and a BBV replication origin. In addition, the recombinant vector may comprise a promoter derived from the genome of a mammalian cell (e.g., metallothionein promoter) or a promoter derived from a mammalian virus (e.g., adenovirus late promoter, vaccinia virus 7.5K promoter, SV40 promoter, cytomegalovirus promoter, and HSV-tk promoter), and generally has a polyadenylation sequence as a transcription termination sequence.

The recombinant vector may comprise a selection marker. The selection marker serves to select a transformant (host cell) transformed with the vector, and since only cells expressing the selection marker can survive in the medium treated with the selection marker, it is possible to select transformed cells. Representative examples of the selection marker include, but are not limited to, ampicillin, kanamycin, streptomycin, and chloramphenicol.

A transformant may be produced by inserting the recombinant vector into a host cell, and the transformant may be obtained by introducing the recombinant vector into an appropriate host cell. The host cell is a cell capable of stably and continuously cloning or expressing the expression vector, and any host cell known in the art may be used.

Where the vector is transformed into prokaryotic cells to generate recombinant microorganisms, examples of host cells that may be used include, but are not limited to, *E. coli* sp. strains such as *E. coli* DH5α, *E. coli* JM109, *E*. *coli* BL21, *E*. *coli* RR1, *E*. *coli* LE392, *E*. *coli* B*, E. coli* X 1776, *E. coli* W3110, and *E. coli* XL1-Blue, *Corynebacterium* sp. strains, *Bacillus* sp. strains such as *Bacillus subtilis* and *Bacillus thuringiensis,* and various Enterobacteriaceae strains such as *Salmonella typhimurium, Serratia marcescens,* and *Pseudomonas* species.

Where the vector is transformed into eukaryotic cells to generate recombinant microorganisms, examples of host cells that may be used include, but are not limited to, yeast (e.g., *Saccharomyces cerevisiae*), insect cells, and plant cells and animal cells, such as Sp2/0, CHO K1, CHO DG44, PER.C6, W138, BHK, COS7, 293, HepG2, Huh7, 3T3, RIN, and MDCK cell lines.

As used in the present invention, the term "transformation" refers to a phenomenon in which external DNA is introduced into a host cell, thereby artificially causing genetic changes, and the term "transformant" refers to a host cell into which external DNA has been introduced and in which the expression of the gene of interest is stably maintained.

The transformation may be performed using a suitable vector introduction technique selected depending on the host cell, so that the gene of interest or a recombinant vector comprising the same may be expressed in the host cell. For example, introduction of the vector may be performed by electroporation, heat-shock, calcium phosphate (CaPO₄) precipitation, calcium chloride (CaCl₂) precipitation, microinjection, polyethylene glycol (PEG) method, DEAE-dextran method, cationic liposome method, lithium acetate-DMSO method, or any combination thereof, without being limited thereto. As long as the transformed gene may be expressed in the host cell, it may be inserted into the chromosome of the host cell, or may exist extrachromosomally, without being limited thereto.

The transformant may include a cell transfected, transformed, or infected with the recombinant vector of the present invention *in vivo* or *in vitro,* and may be used in the same sense as a recombinant host cell, a recombinant cell, or a recombinant microorganism.

Genes inserted into the recombinant vector for transformation according to the present invention may be substituted into a host cell such as a *Corynebacterium* sp. microorganism by homologous recombination crossover.

According to one embodiment of the present invention, the host cell may be *Corynebacterium glutamicum,* for example, *Corynebacterium glutamicum* ATCC 13032, *Corynebacterium glutamicum* ATCC 13761, or the like, without being limited thereto.

According to one embodiment of the present invention, the enzyme that converts 4-hydroxybutyrate to 4-hydroxybutyraldehyde may be carboxylic acid reductase.

The carboxylic acid reductase is an enzyme that catalyzes a reaction that reduces the carboxyl group of 4-hydroxybutyrate, a precursor of 1,4-butanediol, and may be a polypeptide encoded by the car gene and having carboxylic acid reductase activity, without being limited thereto.

According to one embodiment of the present invention, the enzyme that converts 4-hydroxybutyrate to 4-hydroxybutyraldehyde may be encoded by the nucleotide sequence of SEQ ID NO: 12. The gene encoding the enzyme that converts 4-hydroxybutyrate to 4-hydroxybutyraldehyde may essentially comprise or consist of a sequence having 80% homology, preferably 85% homology, more preferably 90% homology, most preferably 95%, 98%, or 99% homology to the nucleotide sequence set forth in SEQ ID NO: 12, in consideration of codon degeneracy, and may retain the original function.

The homology refers to the degree of identity between two given sequences, and may be determined by a known method, such as a method using a known computer algorithm or a method of comparing sequences by a Southern hybridization experiment.

According to one embodiment of the present invention, the enzyme that converts 4-hydroxybutyraldehyde to 1,4-butanediol may be aldehyde reductase YqhD or alcohol dehydrogenase YqhD.

The aldehyde reductase YqhD is an enzyme that removes oxygen from 4-hydroxybutyraldehyde, and may be a polypeptide encoded by the yqhD gene and having aldehyde reductase YqhD activity, without being limited thereto.

According to one embodiment of the present invention, the enzyme that converts 4-hydroxybutyraldehyde to 1,4-butanediol may be encoded by the nucleotide sequence of SEQ ID NO: 5. The gene encoding the enzyme that converts 4-hydroxybutyraldehyde to 1,4-butanediol may essentially comprise or consist of a sequence having 80% homology, preferably 85% homology, more preferably 90% homology, most preferably 95%, 98%, or 99% homology to the nucleotide sequence set forth in SEQ ID NO: 5, in consideration of codon degeneracy, and may retain the original function.

The *Corynebacterium glutamicum* mutant strain for producing 1,4-butanediol according to the present invention may include a metabolic pathway for producing 1,4-butanediol from glutamate. A typical pathway for producing 1,4-butanediol from glutamate sequentially involves an enzymatic reaction that converts glutamate to 4-aminobutyric acid, an enzymatic reaction that converts 4-aminobutyric acid to succinate semialdehyde, an enzymatic reaction that converts succinate semialdehyde to 4-hydroxybutyrate, and an enzymatic reaction that converts 4-hydroxybutyrate to 1,4-butanediol.

According to one embodiment of the present invention, the *Corynebacterium glutamicum* mutant strain may further express at least one of: an enzyme that converts glutamate into 4-aminobutyric acid; an enzyme that converts 4-aminobutyric acid to succinate semialdehyde; and an enzyme that converts succinate semialdehyde to 4-hydroxybutyrate.

According to one embodiment of the present invention, the mutant strain may be one transformed with the vector further comprising at least one of: a gene encoding an enzyme that converts glutamate to 4-aminobutyric acid; a gene encoding an enzyme that converts 4-aminobutyric acid to succinate semialdehyde; and a gene encoding an enzyme that converts succinate semialdehyde to 4-hydroxybutyrate.

The enzyme that converts glutamate to 4-aminobutyric acid, the enzyme that converts 4-aminobutyric acid to succinate semialdehyde, and the enzyme that converts succinate semialdehyde to 4-hydroxybutyrate, or the genes encoding the same, may be derived from microorganisms or bacteria known in the art, and are not particularly limited.

According to one embodiment of the present invention, the enzyme that converts glutamate to 4-aminobutyric acid may be glutamate decarboxylase.

The enzyme that converts glutamate to 4-aminobutyric acid may be derived from *Lactobacillus.* Preferably, it may be encoded by the gadB gene derived from *Lactobacillus senmaizukei,* which is active at pH 7.

According to one embodiment of the present invention, the glutamate decarboxylase may be encoded by the nucleotide sequence of SEQ ID NO: 2. The gene encoding the glutamate decarboxylase may essentially comprise or consist of a sequence having 80% homology, preferably 85% homology, more preferably 90% homology, most preferably 95%, 98%, or 99% homology to the nucleotide sequence set forth in SEQ ID NO: 2, in consideration of codon degeneracy, and may retain the original function.

According to one embodiment of the present invention, the enzyme that converts 4-aminobutyric acid to succinate semialdehyde may be 4-aminobutyrate aminotransferase.

The enzyme that converts the above 4-aminobutyric acid to succinate semialdehyde may be encoded by the gabT gene derived from *E. coli* or *Corynebacterium glutamicum,* without being limited thereto.

According to one embodiment of the present invention, the 4-aminobutyrate aminotransferase may be encoded by the nucleotide sequence of SEQ ID NO: 4 or SEQ ID NO: 33. The gene encoding the 4-aminobutyrate aminotransferase may essentially comprise or consist of a sequence having 80% homology, preferably 85% homology, more preferably 90% homology, most preferably 95%, 98%, or 99% homology to the nucleotide sequence set forth in SEQ ID NO: 4 or SEQ ID NO: 33, in consideration of codon degeneracy, and may retain the original function.

According to one embodiment of the present invention, the enzyme that converts succinate semialdehyde to 4-hydroxybutyrate may be the same aldehyde reductase YqhD or alcohol dehydrogenase YqhD as the enzyme that converts 4-hydroxybutyraldehyde to 1,4-butanediol.

The enzyme that converts succinate semialdehyde to 4-hydroxybutyrate may be encoded by the yqhD gene derived from *E. coli,* without being limited thereto.

According to one embodiment of the present invention, the aldehyde reductase YqhD may be encoded by the nucleotide sequence of SEQ ID NO: 5. The gene encoding the aldehyde reductase YqhD may essentially comprise or consist of a sequence having 80% homology, preferably 85% homology, more preferably 90% homology, most preferably 95%, 98%, or 99% homology to the nucleotide sequence set forth in SEQ ID NO: 5, in consideration of codon degeneracy, and may retain the original function.

Meanwhile, in the 1,4-butanediol production pathway, a side reaction in which succinate semialdehyde is converted to succinate may occur. Therefore, in the present invention, in order to minimize the occurrence of the side reaction in the *Corynebacterium glutamicum* mutant strain for producing 1,4-butanediol, the enzyme involved in the conversion of succinate semialdehyde to succinate may be weakened or inactivated.

According to one embodiment of the present invention, the *Corynebacterium glutamicum* mutant strain may be deficient in the gabD gene encoding succinate-semialdehyde dehydrogenase. For example, the gabD gene may comprise the nucleotide sequence of SEQ ID NO: 1, without being limited thereto.

In addition, in order to minimize the production of by-products such as acetate and lactate in the production of 1,4-butanediol, in the present invention, the enzyme involved in the metabolic pathway for producing acetate or lactate in the *Corynebacterium glutamicum* mutant strain for producing 1,4-butanediol may be weakened or inactivated.

According to one embodiment of the present invention, the *Corynebacterium glutamicum* mutant strain may be deficient in the ldhA gene encoding lactate dehydrogenase A in the metabolic pathway for producing a lactate by-product. For example, the ldhA gene may comprise the nucleotide sequence of SEQ ID NO: 29, without being limited thereto.

In addition, according to one embodiment of the present invention, the *Corynebacterium glutamicum* mutant strain may be deficient in the poxB gene encoding pyruvate oxidase in the metabolic pathway for producing an acetate by-product. For example, the poxB gene may comprise the nucleotide sequence of SEQ ID NO: 30, without being limited thereto.

According to one embodiment of the present invention, the *Corynebacterium glutamicum* mutant strain may be deficient in at least one of the gabD gene, the ldhA gene, and the poxB gene.

Thus, the *Corynebacterium glutamicum* mutant strain according to the present invention has the ability to produce 1,4-butanediol by comprising the pathway for producing 1,4-butanediol from 4-hydroxybutyrate, and may be used as a composition for producing 1,4-butanediol using this characteristic.

Another aspect of the present invention provides a method for producing 1,4-butanediol, comprising steps of: culturing the above-described *Corynebacterium glutamicum* mutant strain; and recovering 1,4-butanediol from the mutant strain or a medium in which the mutant strain has been cultured.

The method for culturing the mutant strain of the present invention may be performed using a suitable medium and culture conditions known in the art, and any person skilled in the art may easily adjust and use the medium and the culture conditions. Specifically, the medium may be a liquid medium, without being limited thereto. Examples of the culturing method include, but are not limited to, batch culture, continuous culture, fed-batch culture, or a combination thereof.

According to one embodiment of the present invention, the medium should meet the requirements of a specific strain in a proper manner, and may be appropriately modified by a person skilled in the art. For culture media for *Corynebacterium glutamicum,* reference may be made to, but not limited to, a known document (Manual of Methods for General Bacteriology, American Society for Bacteriology, Washington D.C., USA, 1981).

According to one embodiment of the present invention, the medium may contain various carbon sources, nitrogen sources, and trace element components.

Examples of carbon sources that may be used include: sugars and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These substances may be used individually or as a mixture, without being limited thereto. In the present invention, glucose is preferably used as a carbon source for producing 1,4-butanediol.

Examples of nitrogen sources that may be used include peptone, yeast extract, meat extract, malt extract, corn steep liquor, soybean meal, urea, or inorganic compounds such as ammonium sulfate, ammonium chloride, ammonium phosphate, ammonium carbonate, and ammonium nitrate. The nitrogen sources may also be used individually or as a mixture, without being limited thereto.

Examples of phosphorus sources that may be used include, but are not limited to, potassium dihydrogen phosphate or dipotassium hydrogen phosphate or the corresponding sodium-containing salts. In addition, the culture medium may contain, but is not limited to, metal salts such as magnesium sulfate or iron sulfate, which are required for growth. In addition, the culture medium may contain essential growth substances such as amino acids and vitamins. Moreover, suitable precursors may be used in the culture medium. The medium or individual components may be added to the culture medium batchwise or in a continuous manner by a suitable method during culturing, without being limited thereto.

According to one embodiment of the present invention, the pH of the culture medium may be adjusted by adding compounds such as ammonium hydroxide, potassium hydroxide, ammonia, phosphoric acid and sulfuric acid to the microorganism culture medium in an appropriate manner during the culturing. In addition, during the culturing, foaming may be suppressed using an anti-foaming agent such as a fatty acid polyglycol ester. Additionally, to keep the culture medium in an aerobic condition, oxygen or an oxygen-containing gas (for example, air) may be injected into the culture medium. The temperature of the culture medium may be generally 20°C to 45°C, for example, 25°C to 40°C. The culturing may be continued until a desired amount of a useful substance is produced. For example, the culturing time may be 10 hours to 160 hours.

According to one embodiment of the present invention, in the step of recovering 1,4-butanediol from the cultured mutant strain or the culture medium containing the same, the produced 1,4-butanediol may be collected or recovered from the medium using a suitable method known in the art depending on the culture method. Examples of a method that may be used to recover the produced 1,4-butanediol include, but are not limited to, centrifugation, filtration, extraction, spraying, drying, evaporation, precipitation, crystallization, electrophoresis, fractional dissolution (e.g., ammonium sulfate precipitation), chromatography (e.g., ion exchange, affinity, hydrophobicity and size exclusion), and the like.

According to one embodiment of the present invention, the step of recovering 1,4-butanediol may be performed by centrifuging the culture medium at a low speed to remove biomass and separating the obtained supernatant through ionexchange chromatography.

According to one embodiment of the present invention, the step of recovering 1,4-butanediol may include a process of purifying 1,4-butanediol.

### Mode for Invention

Hereinafter, the present invention will be described in more detail through examples. The objects, features, and advantages of the present invention will be readily understood through the following examples. The present invention is not limited to the examples described herein and may be embodied in other forms. The examples described herein are provided so that the spirit of the present invention can be sufficiently conveyed to those skilled in the art. Therefore, the present invention should not be construed as being limited by the following examples.

### Example 1. Construction of Recombinant Strain for Production of 4-Hydroxybutyrate

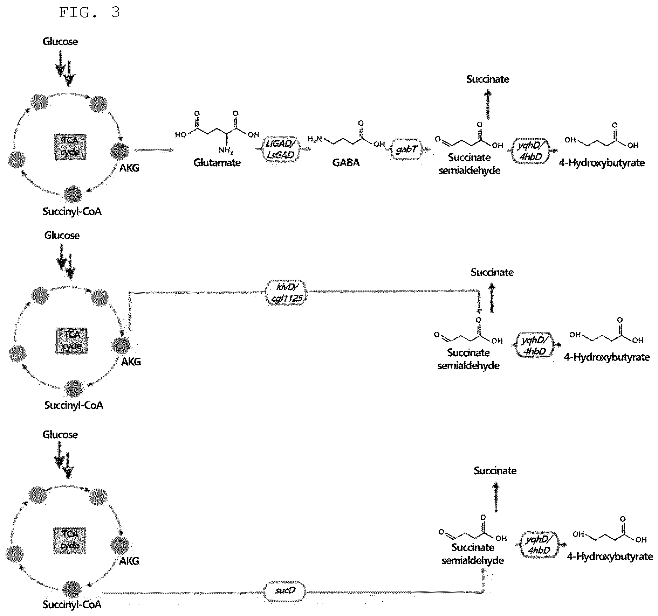

Three pathways (1: glutamate-based metabolic pathway, 2: alpha-ketoglutarate-based metabolic pathway, and 3: succinyl-CoA-based metabolic pathway) shown in FIG. 3 were designed as metabolic pathways for the production of 4-hydroxybutyrate (4HB), a key precursor of 1,4-butanediol (14BDO).

Before introducing the above metabolic pathways, in order to prevent a side reaction in which succinate semialdehyde converts to succinate, the gabD gene (SEQ ID NO: 1) present in *Corynebacterium glutamicum* ATCC 13032 was first deleted using the primers shown in Table 1 below, thereby constructing the recombinant *Corynebacterium* strain C. *glutamicum* ATCC 13032 *ΔgabD2.*

**[Table 1]**

| Primer | SEQ ID NO. | Sequence |
|---|---|---|
| pK19mobsacBGabDf -F | 34 | 5'- cctgcaggcgatctccgtgatcgaatcc -3' |
| pK19mobsacBGabDf -R | 35 | 3'- gctcatgtgttctagattttagcccaccttctggtg - 5' |
| pK19mobsacBGabDb -F | 36 | 5'- tggctaaaatctaga acacatgagctgtccggtga - 3' |
| pK19mobsacBGabDb -R | 37 | 3'- gaattccggggtggcagggttaactc -5' |

Subsequently, the above-constructed metabolic pathways were introduced into *C*. *glutamicum* ATCC 13032 *ΔgabD2,* and plasmids were constructed to compare which metabolic pathway was most efficient. Gene information and primer information are shown in Tables 2 and 3 below.

**[Table 2]**

| Plasmid | Characteristics | Metabolic pathway |
|---|---|---|
| pCES208H30LlgadgabTyqhD | pCES208 derivative; P _{H30}, *Lactobacillus lactis gadB, E. coli gabT, E. coli yqhD*; Km^{r} | 1 |
| pCES208H30LlgadgabT4hbD | pCES208 derivative; P _{H30}, *Lactobacillus lactis gadB, E. coli gabT, C. kluyveri 4hbD;* Km^{r} | |
| pCES208H30LsgadgabTyqhD | pCES208 derivative; P _{H30}, *Lactobacillus senmaizukei gadB, E. coli gabT, E. coli yqhD*; Km^{r} | |
| pCES208H30LsgadgabT4hbD | pCES208 derivative; P _{H30}, *Lactobacillus senmaizukei gadB, E. coli gabT, C. kluyveri 4hbD;* Km^{r} | |
| pCES208H30kivDyqhD | pCES208 derivative; P _{H30}, *Lactobacillus lactis kivD, E. coli yqhD*; Km^{r} | 2 |
| pCES208H30kivD4hbD | pCES208 derivative; P _{H30}, *Lactobacillus lactis kivD, C. kluyveri 4hbD;* Km^{r} | |
| pCES208H30cgl1125yqhD | pCES208 derivative; P _{H30}, *C. glutamicum cgl1125, E. coli yqhD;* Km^{r} | |
| pCES208H30cgl11254hbD | pCES208 derivative; P _{H30}, *C. glutamicum cgl1125, C. kluyveri 4hbD;* Km^{r} | |
| pCES208H30sucDyqhD | pCES208 derivative; P _{H30}, *C. kluyveri sucD, E. coli yqhD;* Km^{r} | 3 |
| pCES208H30sucD4hbD | pCES208 derivative; P _{H30}, *C. kluyveri sucD, C. kluyveri 4hbD;* Km^{r} | |
| Lsgad : Codon-optimized (*C*. *glutamicum-based*) *Lactobacillus senmaizukei gadB* gene sequence, SEQ ID NO: 2 | | |
| Llgad : Codon-optimized (*C. glutamicum*-based) *Lactococcus lactis gadB* gene sequence, SEQ ID NO: 3 | | |
| gabT : *E. coli* gabT, SEQ ID NO: 4 | | |
| yghD : *E. coli* yqhD, SEQ ID NO: 5 | | |
| 4hdD : *Clostridium kluyveri* 4hbD, SEQ ID NO: 6 | | |
| *kivD : Lactobacillus lactis kivD,* SEQ ID NO: 7 | | |
| Cgl1125 : *C. gutamicum* Cgl1125, SEQ ID NO: 8 | | |
| *sucD : C. kluyveri sucD,* SEQ ID NO: 9 | | |

**[Table 3]**

| Primer | SEQ ID NO. | Sequence (5'→3' ) |
|---|---|---|
| Llgad-F-BamHI | 38 | aaaaaggatccatgttatacggaaaagaaaa |
| Llgad-R-SpeI | 39 | aaaaaactagtttagtgagtaaatccatatg |
| Lsgad-F-BamHI | 40 | aaaaaggtaccatgagtaaaa acgatcagg |
| Lsgad-R-SpeI | 41 | aaaaaactagtctaacgaacagtcgtcttg |
| GabT-F-SpeI | 42 | aaaaaactagtaggagatatacatatgaacagcaat aaagag |
| GabT-R-XbaI | 43 | aaaaatctagactactgcttcgcctcatc |
| KivD-F-SpeI | 44 | aaaaaactagtatgtatacagtaggagattac |
| KivD-R-SbfI | 45 | aaaaacctgcaggttatgatttattttgttcag |
| Cgl1125-F-SpeI | 46 | aaaaaactagtaggagatatacatatgctacaactg gggcttagg |
| Cgl1125-R-SbfI | 47 | aaaaacctgcaggttaagcctcgaaagcctcgtc |
| SucD-F-BamHI | 48 | aaaaaggatccatgagtaatgaagtatctat |
| SucD-R-MfeI | 49 | aaaaacaattgttatccccatatttcctcat |
| YqhD-F-MFeI | 50 | aaaaacaattgaggagatatacatatgttacgcgat aaatttattc |
| YqhD-F-SbfI | 51 | aaaaacctgcaggaggagatatacatatgttacgcg ataaatttattc |
| YqhD-F-XbaI | 52 | aaaaatctagaaggagatatacatatgttacgcgataaa tttattc |
| YqhD-R-SbfI | 53 | aaaaacctgcaggctacccccgtccaaactcc |
| YqhD-R-NdeI | 54 | aaaaacatatgctacccccgtccaaactcc |
| 4hbD-F-SbfI | 55 | aaaaacctgcaggaggagatatacatatgaagttattaaaattgg |
| 4hbD-F-XbaI | 56 | aaaaatctagaaggagatatacatatgaagttattaaaattgg |
| 4hbD-R-NdeI | 57 | aaaaacatatgttaatataactttttatatgtg |
| 4hbD-R-NotI | 58 | aaaaagcggccgcttaatataactttttatatgtg |
| 4hbD-R-SbfI | 59 | aaaaacctgcaggttaatataactttttatatgtg |

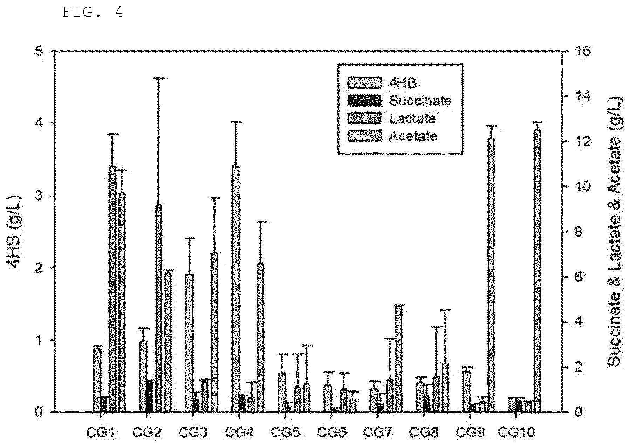

Each plasmid was transformed into *C*. *glutamicum ΔgabD2* which was then flask-cultured, and the amount of 4HB produced was checked. The culture conditions were as described in Sohn et al., ACS Sustainable Chem. Eng. 2021, 9, 6, 2523-2533. The amount produced is graphically shown in FIG. 4.

In FIG. 4, CG1 represents *C*. *glutamicum ΔgabD2* harboring pCES208H30 LlgadgabTyqhD; CG2 represents *C*. *glutamicum ΔgabD2* harboring pCES208H30 LlgadgabT4hbD; CG3 represents *C*. *glutamicum ΔgabD2* harboring pCES208H30 LsgadgabTyqhD; CG4 represents *C*. *glutamicum ΔgabD2* harboring pCES208H30 LsgadgabT4hbD; CG5 represents *C*. *glutamicum ΔgabD2* harboring pCES208H30 kivDyqhD; CG6 represents *C*. *glutamicum ΔgabD2* harboring pCES208H30 kivD4hbD; CG7 represents *C*. *glutamicum ΔgabD2* harboring pCES208H30 cgl1125yqhD; CG8 represents *C*. *glutamicum ΔgabD2* harboring pCES208H30 cgl11254hbD; CG9 represents *C*. *glutamicum ΔgabD2* harboring pCES208H30 sucDyqhD; and CG10 represents *C*. *glutamicum ΔgabD2* harboring pCES208H30 sucD4hbD.

### Example 2. Screening of Metabolic Pathways for Conversion of 4-Hydroxybutyrate to 1,4-Butanediol

The downstream pathways involved in 14BDO biosynthesis can be broadly divided into two pathways: the CoA-dependent pathway and the CoA-independent pathway. Two systems (pBL712H30YqhDCkCat2Bld and pBL712H30YqhDCkCat2PduP) were screened as CoA-dependent systems, and 14 systems (pCES208H30CARpptase + pBL712H30YqhD, CAR1 to CAR14 of SEQ ID NOs: 10 to 23) were screened as CoA-independent systems. For this purpose, the plasmids below were cloned and each primer is described below.

**[Table 4]**

| Plasmid | Description |
|---|---|
| pCES208H30CAR1pptase | pCES208 derivative; P_{H30}, *M. smegmatis CAR (I7**FIG7**), B. subtils pptase;* Km^{r} |
| pCES208H30CAR2pptase | pCES208 derivative; P_{H30}, *M. abscessus CAR (B1MCR9), B. subtils pptase;* Km^{r} |
| pCES208H30CAR3pptase | pCES208 derivative; P_{H30}, *M. abscessus CAR (B1MLD7), B. subtils pptase;* Km^{r} |
| pCES208H30CAR4pptase | pCES208 derivative; P_{H30}, *M. smegmatis CAR (AOR484), B. subtils pptase;* Km^{r} |
| pCES208H30CAR5pptase | pCES208 derivative; P_{H30}, *M. smegmatis CAR (B1MDX4), B. subtils pptase;* Km^{r} |
| pCES208H30CAR6pptase | pCES208 derivative; P_{H30}, *M. smegmatis CAR (AOQWI7), B. subtils pptase;* Km^{r} |
| pCES208H30CAR7pptase | pCES208 derivative; P_{H30}, *M. smegmatis CAR (B1MCS0), B. subtils pptase;* Km^{r} |
| pCES208H30CAR8pptase | pCES208 derivative; P_{H30}, *M. smegmatis CAR (I7GER2), B. subtils pptase;* Km^{r} |
| pCES208H30CAR9pptase | pCES208 derivative; P_{H30}, *M. marinum CAR (B2HN69), B. subtils pptase;* Km^{r} |
| pCES208H30CAR10pptase | pCES208 derivative; P_{H30}, *M. marinum CAR (B2HE95), B. subtils pptase;* Km^{r} |
| pCES208H30CAR11pptase | pCES208 derivative; P_{H30}, *M. paratuberculosis K-10 CAR (Q741P9), B. subtils pptase;* Km^{r} |
| pCES208H30CAR12pptase | pCES208 derivative; P_{H30}, *Nocardia brasiliensis ATCC19296 CAR (A0A034UK40), B. subtils pptase;* Km^{r} |
| pCES208H30CAR13pptase | pCES208 derivative; P_{H30}, *Nocardia brasiliensis ATCC19296 CAR (A0A034UFC8), B. subtils pptase;* Km^{r} |
| pCES208H30CAR14pptase | pCES208 derivative; P_{H30}, *M. phlei* CAR, *B. subtils* pptase; Km^{r} |
| pBL712H30YqhDCkCat2StPdu P | pBL712 derivative; P_{H30}, *C*. *kluyveri cat2, Salmonella enterica pduP, Escherichia coli yqhD;* Sp^{r} |
| pBL712H30YqhDCkCat2Bld | pBL712 derivative; P_{H30}, *C. kluyveri cat2, codon-optimized C. saccharoperbutylacetonicum bld L273T, Escherichia coli yqhD;* Sp^{r} |
| pBL712H30YqhD | pBL712 derivative; P_{H30}, *E*. *coli yghD;* Sp^{r} |
| pptase : *Bacillus subtils* pptase (SEQ ID NO: 24) | |
| pduP : Salmonella enterica pduP (SEQ ID NO: 26) | |
| Bld : *Codon-optimized C*. *saccharoperbutylacetonicum bld L273T* (*Corynebacterium glutamicum-based)* (SEQ ID NO: 27) | |
| Cat2 : *C. kluyveri cat2* (SEQ ID NO: 28) | |

**[Table 5]**

| Primer | SEQ ID NO. | Sequence (5'→3' ) |
|---|---|---|
| YqhD-F-EcoRI | 60 | aaaaaggatccaggagatatacatatgaacaactttaatctgcaca ccc |
| YqhD-R-KpnI | 61 | aaaaaagatctatgtatatctcctttagcgggcggcttcgtatata c |
| StPduP-F-KpnI | 62 | ggtaccaggagatatacatatgaatacttctgaactcgaaacc |
| StPduP-R-BamHI | 63 | ggatccttagcgaatagaaaagccgttgg |
| Bld-F-KpnI | 64 | ggtaccaggagatatacatatgattaaagacacgctag |
| Bld-R-BamHI | 65 | ggatccttaaccggcgagtacacatc |
| CkCat2-F-SbfI | 66 | cctgcaggaggagatatacatatggagtgggaagagatata |
| CkCat2-R-HindIII | 67 | aagcttctaaaatctctttttaaattc |

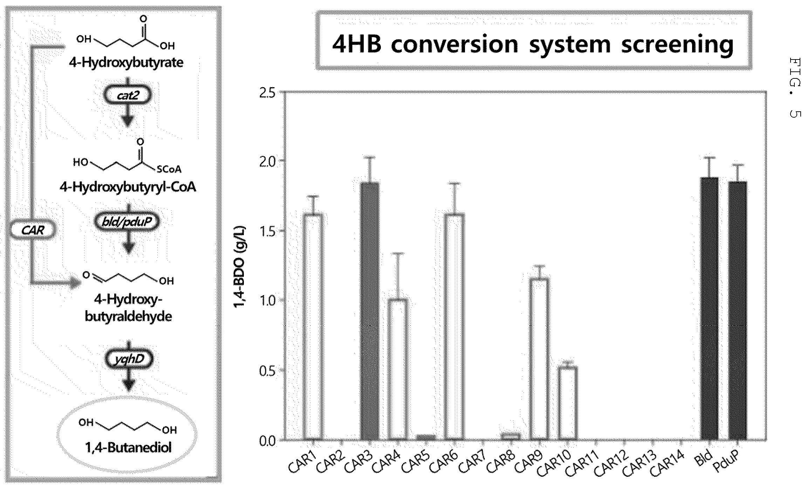

Thereafter, each plasmid was transformed into C. *glutamicum ΔgabD2* which was then fed with 2 g/L of Na4HB was fed and flask-cultured for 120 hours. The results are graphically shown in FIG. 5. As a result, three systems, pCES208H30CAR3pptase + pBL712H30YqhD (CoA-independent), pBL712H30YqhDCat2Bld, and pBL712H30YqhDCat2StPduP (CoA-dependent), showed the highest conversion from 4HB to 14BDO. Subsequent experiments were conducted using CAR3 selected as the CoA-independent pathway and Bld selected as the CoA-dependent pathway.

### Example 3. Production of 14BDO from Glucose Using Recombinant Corynebacterium Strain

### 3-1. Construction of Recombinant Corynebacterium Strains

Prior to producing 14BDO, additional metabolic engineering improvement of *C*. *glutamicum ΔgabD2* was performed for more efficient production. Since a large amount of by-products such as acetate and lactate were produced in 4HB production, the metabolic pathways for producing acetate (poxB) and lactate (ldhA) by-products existing in *Corynebacterium* were removed to develop a recombinant *Corynebacterium* strain (*C. glutamicum* ATCC 13032 *ΔldhA ΔpoxB ΔgabD2)* (ldhA gene sequence = SEQ ID NO: 29; and poxB gene sequence = SEQ ID NO: 30).

To construct each gene deletion pK19mobsacB (NEB)-based vector, 500-bp homologous regions upstream and downstream of each gene were amplified through overlap PCR to produce a 1,000-bp PCR product, which was then cloned into the ldhA (HindIII/EcoRI), poxB (HindIII/EcoRI), and gabD (SbfI/EcoRI) sites: pK19mobsacBLdhA, pK19mobsacBPoxB, and pK19mobsacBGabD. The primers used here are shown in Table 6 below.

**[Table 6]**

| Primer | SEQ ID NO. | Sequence (5'→3' ) |
|---|---|---|
| pK19mobsacBLdhAf -F | 68 | aagcttagtgaccatcggcggagtgttcg |
| pK19mobsacBLdhAf -R | 69 | ggggcagatgtctagagatgcgttattttccttcac |
| pK19mobsacBLdhAb -F | 70 | cgcatctctagacatctgcccctttacaaatcc |
| pK19mobsacBLdhAb -R | 71 | gaattccctgccatccagtcggcatac |
| pK19mobsacBPoxBf -F | 72 | gcttgagaatttcggcgtgctcgtc |
| pK19mobsacBPoxBf -R | 73 | gggatcgaaaatctttggcgcctagttggcg |
| pK19mobsacBPoxBb -F | 74 | cgccaaagattttcgatcccacttcctgatttc |
| pK19mobsacBPoxBb -R | 75 | gaattcaaaacagccaggttagcagcc |

Thereafter, the above-constructed C. *glutamicum* ATCC 13032 *ΔldhA ΔpoxB ΔgabD2* strain was transformed with each of the 4HB pathways (Example 1) and the 4HB conversion pathways (Example 2), thereby constructing the final recombinant strains. The 14BDO production of each of the produced recombinant strains was compared. The 14BDO productivity was compared between the strains using the CoA-dependent pathway and the strains using the CoA-independent pathway.

### 3-2. 14BDO Productivity of Strains Using CoA-Dependent Pathway

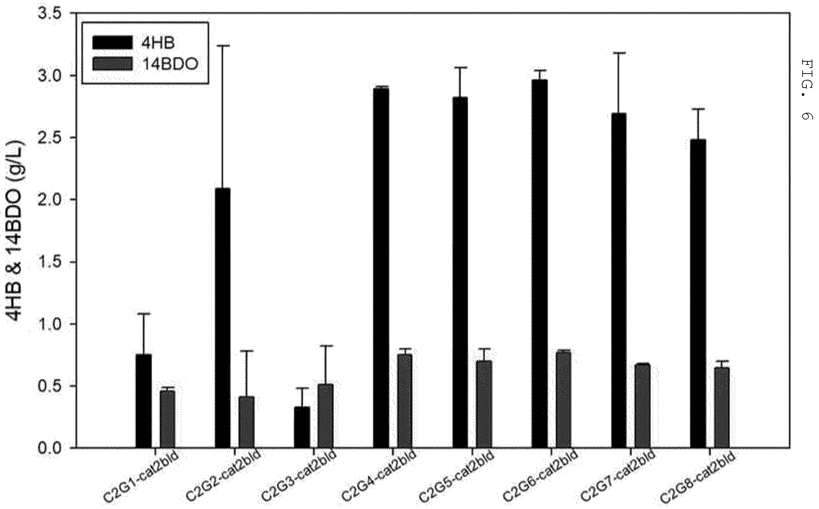

FIG. 6 shows the results of analyzing the 14BDO productivity of the strains that use the CoA-dependent pathway.

In FIG. 6, C2G1-cat2bld represents (*C. glutamicum ΔldhA ΔpoxB ΔgabD2* harboring pCES208H30 LsgadgabTyqhD and pBL712H30 yqhDbldcat2), C2G2-cat2bld represents (*C*. *glutamicum ΔldhA ΔpoxB ΔgabD2* harboring pCES208H30 LsgadgabT4hbD and pBL712H30 yqhDbldcat2), C2G3-cat2bld represents *(C. glutamicum ΔldhA ΔpoxB ΔgabD2* harboring pCES208H30 kivDyqhD and pBL712H30 yqhDbldcat2), C2G4-cat2bld represents *(C. glutamicum ΔldhA ΔpoxB ΔgabD2* harboring pCES208H30 kivD4hbD and pBL712H30 yqhDbldcat2), C2G5-cat2bld represents *(C. glutamicum ΔldhA ΔpoxB ΔgabD2* harboring pCES208H30 cgl1125yqhD and pBL712H30 yqhDbldcat2), C2G6-cat2bld represents (*C. glutamicum ΔldhA ΔpoxB ΔgabD2* harboring pCES208H30 cgl11254hbD and pBL712H30 yqhDbldcat2), C2G7-cat2bld represents (*C. glutamicum ΔldhA ΔpoxB ΔgabD2* harboring pCES208H30 sucDyqhD and pBL712H30 yqhDbldcat2), and C2G8-cat2bld represents (*C. glutamicum ΔldhA ΔpoxB ΔgabD2* harboring pCES208H30 sucD4hbD and pBL712H30 yqhDbldcat2).

Among the strains using the CoA-dependent pathway, the C2G6-Cat2Bld strain showed the highest production of 14BDO, which was 0.77 g/L.

### 3-3. 14BDO Productivity of Strains Using CoA-Independent Pathway

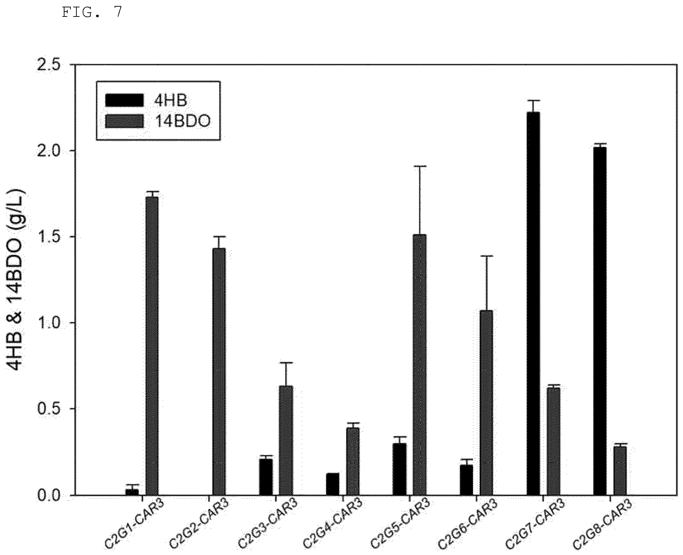

FIG. 7 shows the results of analyzing the 4BDO productivity of the strains that use the CoA-independent pathway.

In FIG. 7, C2G1-CAR3 represents (*C. glutamicum ΔldhA ΔpoxB ΔgabD2* harboring pCES208H30 LsgadgabTyqhD and pBL712H30 CAR3PPTaseyqhD), C2G2-CAR3 represents (*C*. *glutamicum ΔldhA ΔpoxB ΔgabD2* harboring pCES208H30 LsgadgabT4hbD and pBL712H30 CAR3PPTaseyqhD), C2G3-CAR3 represents (*C. glutamicum ΔldhA ΔpoxB ΔgabD2* harboring pCES208H30 kivDyqhD and CAR3PPTaseyqhD), C2G4-CAR3 representns (*C. glutamicum ΔldhA ΔpoxB ΔgabD2* harboring pCES208H30 kivD4hbD and pBL712H30 CAR3PPTaseyqhD), C2G5-CAR3 represents (*C. glutamicum ΔldhA ΔpoxB ΔgabD2* harboring pCES208H30 cgl1125yqhD and pBL712H30 CAR3PPTaseyqhD), C2G6-CAR3 represents (*C. glutamicum ΔldhA ΔpoxB ΔgabD2* harboring pCES208H30 cgl11254hbD and pBL712H30 CAR3PPTaseyqhD), C2G7-CAR3 represents (*C. glutamicum ΔldhA ΔpoxB ΔgabD2* harboring pCES208H30 sucDyqhD and pBL712H30 CAR3PPTaseyqhD), and C2G8-CAR3 represents *(C. glutamicum ΔldhA ΔpoxB ΔgabD2* harboring pCES208H30 sucD4hbD and pBL712H30 CAR3PPTaseyqhD).

Among the strains using the CoA-independent pathway, the C2G1-CAR3 strain showed the highest production of 14BDO, which was 1.73 g/L. The C2G1-CAR3 strain also did not produce any by-products.

### Example 4. Construction of Corynebacterium glutamicum Mutant Strain with Constructed 14BDO Production Pathway

### 4-1. Construction of gadB, gabT, yqhD, car and sfp Gene Introduction Vectors and gabD Deletion Vectors

A pathway for biosynthesizing 1,4-butanediol from glutamate was constructed by introducing the gadB (SEQ ID NO: 2), gabT (SEQ ID NO: 33), yqhD (SEQ ID NO: 5), car (SEQ ID NO: 12), and sfp (SEQ ID NO: 25) genes into *Corynebacterium glutamicum* ATCC 13032.

The chromosomal DNA of *Corynebacterium glutamicum* ATCC 13032 was used as a template and amplified by PCR using each of primers 1 and 2, primers 3 and 4, primers 7 and 8, primers 9 and 10, and primers 11 and 12. pCES208H30LsGAD (ACS Omega. 2022 Aug 23; 7(33): 29106-29115.) DNA containing the gadB gene (derived from *Lactobacillus senmaizukei*) was used as a template and amplified by PCR using primers 5 and 6. The resulting PCR products were amplified by crossover PCR and then inserted into the restriction enzyme sites HindIII and XbaI of the pK19mobSacB vector (ATCC, 87098). The resulting vector was named pk19mobsacB-LsGADgabT.

The chromosomal DNA of *Corynebacterium glutamicum* ATCC 13032 was used as a template and amplified by PCR using each of primers 13 and 14, primers 15 and 16, and primers 19 and 20. The chromosomal DNA of *Escherichia coli* K-12 MG1655 containing the yqhD gene (derived from *Escherichia coli*) was used as a template and amplified by PCR using primers 17 and 18. The resulting PCR products were amplified by crossover PCR and then inserted into the restriction enzyme sites HindIII and XbaI of the pK19mobSacB vector. The resulting vector was named pk19mobsacB-yqhD.

The chromosomal DNA of *Corynebacterium glutamicum* ATCC 13032 was used as a template and amplified by PCR using each of primers 21 and 22, primers 23 and 24, and primers 27 and 28. The *Escherichia coli* expression vector pKE112CAR3pptase (Polymers (Basel). 2019 11(7):1184) containing the car gene (derived from *Mycobacterium abscessus*) was used as a template and amplified by PCR using primers 25 and 26. The resulting PCR products were amplified by crossover PCR and then inserted into the restriction enzyme sites HindIII and XbaI of the pK19mobSacB vector. The resulting vector was named pk19mobsacB-car.

To insert the sfp gene required to activate the enzymatic activity of the car gene, the chromosomal DNA of *Corynebacterium glutamicum* ATCC 13032 was used as a template and amplified by PCR using each of primers 29 and 30, primers 31 and 32, and primers 35 and 36. The *Escherichia coli* expression vector pKE112CAR3pptase (Polymers (Basel). 2019 11(7):1184) containing the sfp gene (derived from *Bacillus subtilis*) was used as a template and amplified by PCR using primers 33 and 34. The resulting PCR products were amplified by crossover PCR and then inserted into the restriction enzyme sites HindIII and XbaI sites of the pK19mobSacB vector. The resulting vector was designated pk19mobsacB-sfp.

To delete the gabD gene (SEQ ID NO: 1), the chromosomal DNA of *Corynebacterium glutamicum* ATCC 13032 was used as a template and amplified by PCR using primers 37 and 38, and primers 39 and 40, respectively. The PCR products obtained through this were amplified by crossover PCR and then inserted into the restriction enzyme sites HindIII and XbaI of the pK19mobSacB vector. The resulting vector was named pk19mobsacB-*ΔgabD2*.

Here, the Wizard Genomic DNA Purification Kit (Promega, USA) was used to extract chromosomal DNA from each strain. The primers used for PCR are shown in Table 7 below. Using a thermocycler (TP600, TAKARA BIO Inc., Japan) and a reaction solution containing 100 µM of each deoxynucleotide triphosphate (dATP, dCTP, dGTP, dTTP), 1 pM of oligonucleotide, and 10 ng of template DNA, PCR was performed in the presence of one unit of a PrimeSTAR Max DNA polymerase

### (Takara, Japan)

Using the NEBuilder HiFi DNA Assembly Master Mix (NEB, USA) and the restriction enzymes HindIII (NEB, USA) and XbaI (NEB, USA), the constructed pk19mobsacB-LsGADgabT, pk19mobsacB-yqhD, pk19mobsacB-car, pk19mobsacB-sfp, and pk19mobsacB-*ΔgabD2* vectors were each transformed into *E*. *coli DH5a* (HIT Competent cells^{™}, Cat No. RH618), which was then spread on LB-agar plates containing 50 µg/ml of kanamycin and cultured at 37°C for 24 hours. After the finally formed colonies were isolated and it was checked whether the inserts would be exactly present in the vector, the vector was isolated and used to construct a *Corynebacterium glutamicum* mutant strain into which the pathway for producing 1,4-butanediol from glutamate has been introduced.

**[Table 7]**

| Primer | SEQ ID NO. | Sequence (5'→3') |
|---|---|---|
| Primer 1 | 76 | aacagctatgaccatgattacgccacaaggattacgagctcgttggtga g |
| Primer 2 | 77 | cttcggatctaaacgatctgggcttttaccttcgtttcgc |
| Primer 3 | 78 | cgaaggtaaaagcccagatcgtttagatccgaaggaaaac |
| Primer 4 | 79 | tgatcgtttttactcattgtatgtcctcctggacttcgtg |
| Primer 5 | 80 | tccaggaggacatacaatgagtaaaaacgatcaggaaacg |
| Primer 6 | 81 | ttggcagctaagtagggtttaacgaacagtcgtcttgttc |
| Primer 7 | 82 | gacgactgttcgttaaaccctacttagctgccaattattc |
| Primer 8 | 83 | gtatgagagatcttccacgggtaaaaaatcctttcgtagg |
| Primer 9 | 84 | aaggattttttacccgtggaagatctctcataccgcatcc |
| Primer 10 | 85 | caagggtccggctttttgttagcccaccttctggtgcgcg |
| Primer 11 | 86 | cagaaggtgggctaacaaaaagccggacccttgctttaag |
| Primer 12 | 87 | ggtacccggggatcctctagtgctcatgcagtacctgc |
| Primer 13 | 88 | ctatgaccatgattacgcgggcgatggcggcgaatccg |
| Primer 14 | 89 | ccttcggatctaaacgatctttccttaagtgctgattcgc |
| Primer 15 | 90 | gcgaatcagcacttaaggaaagatcgtttagatccgaagg |
| Primer 16 | 91 | gcagattaaagttgttcattgtatgtcctcctggacttcg |
| Primer 17 | 92 | ccaggaggacatacaatgaacaactttaatctgcacaccc |
| Primer 18 | 93 | tcagaacctgtaggtcttagcgggcggcttcgtatatacg |
| Primer 19 | 94 | cgaagccgcccgctaagacctacaggttctgacaatttaaatctc |
| Primer 20 | 95 | gctcggtacccggggatcctctgggacttcagcaacatcg |
| Primer 21 | 96 | aacagctatgaccatgattacgccatccgacctggccggtgatgg |
| Primer 22 | 97 | agctaagtagggtgagccaagattagcgctgaaaagtage |
| Primer 23 | 98 | gcgctaatcttggctcaccctacttagctgccaattattc |
| Primer 24 | 99 | ggagatcgtttcagtcatgggtaaaaaatcctttcgtagg |
| Primer 25 | 100 | aaggattttttacccatgactgaaacgatctccacagcgg |
| Primer 26 | 101 | gtctgtaatcagcgtcctactacaccaggcccaacagctg |
| Primer 27 | 102 | ttgggcctggtgtagtaggacgctgattacagacgtgtcc |
| Primer 28 | 103 | Ggtacccggggatcctctagtctgctctaaagagcggcgggtgg |
| Primer 29 | 104 | Gaccatgattacgccaagctcgacgcagaaggtgtgatcc |
| Primer 30 | 105 | aattggcagctaagtagggtttagcccaccttctggtgcg |
| Primer 31 | 106 | cagaaggtgggctaaaccctacttagctgccaattattcc |
| Primer 32 | 107 | taaattccgtaaatcttcatgggtaaaaaatcctttcgta |
| Primer 33 | 108 | aaggattttttacccatgaagatttacggaatttatatgg |
| Primer 34 | 109 | tcacggcaaagcgaggtacttataaaagctcttcgtacg |
| Primer 35 | 110 | cgtacgaagagcttttataagtacctcgctttgccgtgac |
| Primer 36 | 111 | ggtacccggggatcctctagcgaagcttgccgtgtgcagg |
| Primer 37 | 112 | tgattacgccaagctgacaacaattgcgccatcgc |
| Primer 38 | 113 | ttgtaaaggggcagatgctattccacatcgaaagtaccgc |
| Primer 39 | 114 | tactttcgatgtggaatagcatctgcccctttacaaatcc |
| Primer 40 | 115 | ggtacccggggatcctctagtttgcggagccttgagatcg |

### 4-2. Construction of Corynebacterium glutamicum Mutant Strain

The pk19mobsacB-LsGADgabT vector constructed in Example 4-1 above was introduced into competent cells of *Corynebacterium glutamicum* ATCC 13032 by electroporation using an electrophorator (BIO-RAD, USA), and then the cells were plated on 2YT KM AGAR medium (containing 2YT AGAR and 30 mg/L kanamycin) and cultured in an incubator at 30°C for 2 days to obtain colonies. Among the colonies in which the first homologous recombination was induced, the colonies confirmed by PCR were cultured in 2YT liquid medium (containing 16 g/L tryptophan, 10 g/L yeast extract, and 5 g/L NaCl) for 12 hours, and then plated on 2YT sucrose AGAR medium (containing 2YT AGAR and 100 g/L sucrose), and the antibiotic marker was removed by the second homologous recombination. The selected colonies were finally analyzed by PCR and sequencing to determine whether the gadB and gabT genes were introduced as intended.

Thereafter, the above-described procedures were sequentially performed using the pk19mobsacB-yqhD, pk19mobsacB-car, pk19mobsacB-sfp and pk19mobsacB-Δ*gabD2* vectors constructed in Example 4-1 above, thereby constructing a *Corynebacterium glutamicum* mutant strain in which the pathway for producing 1,4-butanediol from glutamate has been established by introduction of the gadB, gabT, yqhD, car and sfp genes. The constructed mutant strain was named WB04-0026.

### Example 5. Evaluation of 1,4-Butanediol Productivity of Corynebacterium glutamicum Mutant Strain

The 1,4-butanediol productivity of the *Corynebacterium glutamicum* mutant strain (WB04-0026) expressing the genes of the 14BDO biosynthetic pathway was evaluated in comparison with that of the parent strain *Corynebacterium glutamicum* ATCC 13032.

Each strain was inoculated into a flask medium (containing 10% glucose, 0.25% MgSO₄, 2.5% yeast extract, 0.25% KH₂PO₄, 2.5% (NH₄)₂SO₄, 100 ppm FeSO₄, 2.5% peptone, 100 µg/L biotin, 25 ppm nicotinamide, 25 ppm CPN, and 1% pyridoxine) and cultured at 30°C for 24 hours. After completion of the culturing, the culture was filtered through a 0.45 µm filter, and then the 1,4-butanediol content in the culture was analyzed using high-performance liquid chromatography (HPLC) (Agilent, 1260 infinity II) equipped with a column (Avantor HPLC Column Apollo C18). A 0.1 M phosphate buffer was used as the mobile phase, and the analysis was performed using an RI detector at a temperature of 40°C and a flow rate of 0.8 mL/min for 15 minutes. The results are shown in Table 8 below.

**[Table 8]**

| Strain | 1,4-butanediol (g/L) |
|---|---|
| WB04-0026 | 0.6 |
| ATCC 13032 | 0 |

**As** shown in Table 8 above, it was confirmed that the *Corynebacterium glutamicum* mutant strain (WB04-0026) expressing the genes of the 1,4-butanediol biosynthetic pathway had 1,4-butanediol productivity compared to the wild-type *Corynebacterium glutamicum* (ATCC 13032).

Although the present invention has been described above with reference to preferred embodiments thereof, it will be understood by those skilled in the art that various modifications and changes may be made to the present invention without departing from the spirit and scope of the present invention as set forth in the claims below.

## Claims

1. A *Corynebacterium glutamicum* mutant strain for producing 1,4-butanediol, which expresses: an enzyme that converts 4-hydroxybutyrate to 4-hydroxybutyraldehyde; and an enzyme that converts 4-hydroxybutyraldehyde to 1,4-butanediol.

2. The mutant strain of claim 1, wherein the mutant strain is transformed with a vector comprising: a gene encoding the enzyme that converts 4-hydroxybutyrate to 4-hydroxybutyraldehyde; and a gene encoding the enzyme that converts 4-hydroxybutyraldehyde to 1,4-butanediol.

3. The mutant strain of claim 1, wherein the enzyme that converts 4-hydroxybutyrate to 4-hydroxybutyraldehyde is carboxylic acid reductase.

4. The mutant strain of claim 1, wherein the enzyme that converts 4-hydroxybutyrate to 4-hydroxybutyraldehyde consists of the amino acid sequence of SEQ ID NO: 31.

5. The mutant strain of claim 1, wherein the enzyme that converts 4-hydroxybutyraldehyde to 1,4-butanediol is aldehyde reductase YqhD or alcohol dehydrogenase YqhD.

6. The mutant strain of claim 1, wherein the enzyme that converts 4-hydroxybutyraldehyde to 1,4-butanediol consists of the amino acid sequence of SEQ ID NO: 32.

7. The mutant strain of claim 1, which further expresses at least one of: an enzyme that converts glutamate to 4-aminobutyric acid; an enzyme that converts 4-aminobutyric acid to succinate semialdehyde; and an enzyme that converts succinate semialdehyde to 4-hydroxybutyrate.

8. The mutant strain of claim 1, wherein the mutant strain is one transformed with the vector further comprising at least one of: a gene encoding an enzyme that converts glutamate to 4-aminobutyric acid; a gene encoding an enzyme that converts 4-aminobutyric acid to succinate semialdehyde; and a gene encoding an enzyme that converts succinate semialdehyde to 4-hydroxybutyrate.

9. The mutant strain of claim 7, wherein the enzyme that converts glutamate to 4-aminobutyric acid is glutamate decarboxylase.

10. The mutant strain of claim 7, wherein the enzyme that converts 4-aminobutyric acid to succinate semialdehyde is 4-aminobutyrate aminotransferase.

11. The mutant strain of claim 7, wherein the enzyme that converts the succinate semialdehyde to 4-hydroxybutyrate is aldehyde reductase YqhD or alcohol dehydrogenase YqhD.

12. The mutant strain of claim 1, wherein the mutant strain is deficient in at least one of gabD gene, ldhA gene, and poxB gene.

13. A method for producing 1,4-butanediol, comprising steps of:
culturing the mutant strain of claim 1, 7 or 11; and
recovering 1,4-butanediol from the mutant strain or a medium in which the mutant strain has been cultured.
